# EUROPEAN PATENT SPECIFICATION

(11) **EP 4 231 271 B1**
(45) Date of publication and mention of the grant of the patent: **08.07.2026**
(21) Application number: 23157249.6
(22) Date of filing: 17.02.2023
(51) Int. Cl.: G09B 23/28, A61B 8/00

(54) **METHOD AND SYSTEM FOR GENERATING A SIMULATED MEDICAL IMAGE**
VERFAHREN UND SYSTEM ZUR ERZEUGUNG EINES SIMULIERTEN MEDIZINISCHEN BILDES
PROCÉDÉ ET SYSTÈME DE GÉNÉRATION D'UNE IMAGE MÉDICALE SIMULÉE

(30) Priority: 17.02.2022 CA 3149196
(43) Date of publication of application: 23.08.2023
(73) Proprietor: North America Elevate Healthcare Human Capital Inc., Saint-Laurent, QC H4T 1G6 (CA)
(72) Inventor: DRISCOLL, Christopher, Saint-Laurent, QC H4T 1G6 (CA); FRASER, Dominic, Saint-Laurent, QC H4T 1G6 (CA); LAPIERRE, Maxime, Saint-Laurent, QC H4T 1G6 (CA)
(74) Representative: Kolster Oy Ab

(56) References cited:
- US-A1- 2010 285 438
- US-B2- 9 870 721

## Description

### TECHNICAL FIELD

The present invention relates to the field of medical imaging, and more particularly to the simulation of medical images for training a medical practitioner.

### BACKGROUND

Medical imaging such as ultrasound imaging is widely used in the medical field, notably during surgeries. While the surgeon manipulates a surgical tool within the body of a patient, another healthcare professional manipulates a medical probe such as an ultrasound probe to ensure the tip of the surgical tool continuously appears on the images. The images can help the surgeon estimate the position of the tool within the body of the patient during the surgery.

Systems for simulating surgeries such as interventional cardiology procedures have been developed for training surgeons for example. Some of these systems display simulated ultrasound images of the body to mimic a real surgical procedure. While using the system and moving the medical tool, a surgeon-in-training may select different predefined and fixed views for the displayed simulated ultrasound image in order to visualize the medical tool. However, since an ultrasound beam is thin, the medical tool may not always intersect the simulated ultrasound beam and therefore may not appear in the displayed ultrasound images independently of the selected view. Prior art document US 9870721 shows a device for performing image-guided interventions. An acquired ultrasound image can be displayed and can be manipulated to guide a virtual instrument. Prior art document US 2010/1285438 shows a system configured to control a virtual surgical tool. A target position of the tool may be determined to guide the user.

Therefore, there is a need for a method and system to automatically maintain visibility of surgical tools within medical images.

### SUMMARY

A feature was developed which allows a single learner playing the role of the surgeon to manipulate surgical tools within a medical simulation device while having the field of view automatically adjust in order to allow continued visualization of the tool's distal tip. One application could have the position of an ultrasound beam origin be based on user-selected standardized views while the origin of the beam adjusts to follow the tools. Another application could have both the position and orientation of an ultrasound beam adjust to follow the tools. Additionally, limits can be placed on the positional and orientation adjustments to ensure that the generated ultrasound images remain anatomically relevant (i.e. convey the anatomy consistent with standard medical views).

According to a first broad aspect, there is provided a computer-implemented method for generating a simulated ultrasound image of a manikin part, the computer-implemented method comprising: determining a desirable position for a virtual ultrasound probe based on an actual position of a tip of a medical tool in order for a virtual ultrasound beam emitted by the virtual ultrasound probe to intersect the tip of the medical tool; generating the simulated ultrasound image of the manikin part according to the desirable position of the virtual ultrasound probe, the simulated ultrasound image comprising a representation of the tip of the medical tool and a representation of a region of the manikin part surrounding the tip of the medical tool; and providing the simulated ultrasound image for display.

In one embodiment, the computer-implemented method further comprises determining the actual position of the tip of the medical tool.

In one embodiment, the step of determining the desirable position for the virtual ultrasound probe comprises at least one of determining desirable position coordinates for a reference point located on the virtual ultrasound probe and determining a desirable orientation of the virtual ultrasound probe.

In one embodiment, the step of determining the desirable position for the virtual ultrasound probe comprises determining the desirable orientation of the virtual ultrasound probe, the desirable position coordinates for the reference point located on the virtual ultrasound probe being fixed.

**In** one embodiment, the step of determining the desirable orientation for the virtual ultrasound probe comprises selecting the desirable orientation for the virtual ultrasound probe amongst predefined orientations based on the actual position of the tip of the medical tool.

**In** one embodiment, the step of determining the desirable position for the virtual ultrasound probe comprises determining the desirable position coordinates for the reference point located on the virtual ultrasound probe, the desirable orientation of the virtual ultrasound probe being fixed.

**In** one embodiment, the step of determining the desirable position coordinates for the reference point located on the virtual ultrasound probe comprises selecting the desirable position coordinates amongst predefined position coordinates based on the position of the tip of the medical tool.

In one embodiment, the manikin part comprises an esophagus, the predefined position coordinates being located along the esophagus.

In one embodiment, the step of determining the desirable position for the virtual ultrasound probe comprises determining a desirable position variation for the virtual ultrasound probe.

In one embodiment, the step of determining the desirable position variation for the virtual ultrasound probe comprises at least one of determining a desirable variation in position coordinates for a reference point located on the virtual ultrasound probe and determining a desirable variation in orientation of the virtual ultrasound probe.

According to another broad aspect, there is provided a system for generating a simulated ultrasound image of a manikin part, the system comprising: a probe determining unit for determining a desirable position for a virtual ultrasound probe based on an actual position of a tip of a medical tool in order for a virtual ultrasound beam emitted by the virtual ultrasound probe to intersect the tip of the medical tool; and a simulation engine for generating the simulated ultrasound image of the manikin part according to the desirable position of the virtual ultrasound probe, and providing the simulated ultrasound image for display, the simulated ultrasound image comprising a representation of the tip of the medical tool and a representation of a region of the manikin part surrounding the tip of the medical tool.

In one embodiment, the system further comprises a position sensor for determining the actual position of the tip of the medical tool.

In one embodiment, the probe determining unit is configured for determining the desirable position by determining at least one of desirable position coordinates for a reference point located on the virtual ultrasound probe and a desirable orientation of the virtual ultrasound probe.

In one embodiment, the probe determining unit is configured for determining the desirable position by determining the desirable orientation of the virtual ultrasound probe, the desirable position coordinates for the reference point located on the virtual ultrasound probe being fixed.

In one embodiment, the probe determining unit is configured for selecting the desirable orientation for the virtual ultrasound probe amongst predefined orientations based on the actual position of the tip of the medical tool.

In one embodiment, the probe determining unit is configured for determining the desirable position coordinates for the reference point located on the virtual ultrasound probe, the desirable orientation of the virtual ultrasound probe being fixed.

In one embodiment, the probe determining unit is configured for selecting the desirable position coordinates amongst predefined position coordinates based on the position of the tip of the medical tool.

In one embodiment, the manikin part comprises an esophagus, the predefined position coordinates being located along the esophagus.

In one embodiment, the probe determining unit is configured for determining a desirable position variation for the virtual ultrasound probe.

In one embodiment, the probe determining unit is configured for at least one of determining a desirable variation in position coordinates for a reference point located on the virtual ultrasound probe and determining a desirable variation in orientation of the virtual ultrasound probe.

According to a further broad aspect, there is provided a computer program product for generating a simulated ultrasound image of a manikin part, the computer program product comprising a computer readable memory storing computer executable instructions thereon that when executed by a computer perform the method steps of: determining a desirable position for a virtual ultrasound probe based on an actual position of a tip of a medical tool in order for a virtual ultrasound beam emitted by the virtual ultrasound probe to intersect the tip of the medical tool; generating the simulated ultrasound image of the manikin part according to the desirable position of the virtual ultrasound probe, the simulated ultrasound image comprising a representation of the tip of the medical tool and a representation of a region of the manikin part surrounding the tip of the medical tool; and providing the simulated ultrasound image for display.

It will be understood that the term "position" should be interpreted broadly so as to encompass the position and/or orientation, or a variation in position and/or a variation in orientation. Therefore, a position may be defined by absolute coordinates, a translation vector, rotation angle(s), etc. In one embodiment, the position of a virtual medical probe may refer to the position coordinates of a reference point of the virtual medical probe. The position coordinates may define a position in a 2D space or in a 3D space. The position coordinates may be expressed in a Cartesian coordinate system, a cylindrical coordinate system or the like. In another embodiment, the position of the virtual medical probe may refer to the orientation of the virtual medical probe. In a further embodiment, the position of the virtual medical probe may refer to both position coordinates of the reference point of the virtual medical probe and the orientation of the virtual medical probe. In still another embodiment, the position of the virtual medical probe may refer to a variation in position such as a variation in position coordinates of a reference point of the virtual medical probe and/or a variation in orientation of the virtual medical probe.

### BRIEF DESCRIPTION OF THE DRAWINGS

Further features and advantages of the present invention will become apparent from the following detailed description, taken in combination with the appended drawings, in which:
FIG. 1A is a flow chart illustrating a computer-implemented method for generating a simulated medical image representing a part of manikin and the tip of a medical tool based on the position of a virtual medical probe, in accordance with an embodiment;
FIG. 1B is a flow chart illustrating a computer-implemented method for generating a simulated ultrasound image representing a part of manikin and the tip of a medical tool based on the position of a virtual ultrasound probe, in accordance with an embodiment;
FIG. 2 schematically illustrates a medical tool and a virtual ultrasound probe emitting a virtual ultrasound beam that intersects the tip of the medical tool, in accordance with an embodiment;
FIG. 3A schematically illustrates the rotation of a virtual ultrasound beam virtually emitted by a virtual ultrasound probe about an axis orthogonal to the plane of the virtual ultrasound beam, in accordance with an embodiment;
FIG. 3B schematically illustrate a rotation of the virtual ultrasound beam about an axis contained within the plane of the virtual ultrasound beam, in accordance with an embodiment;
FIG. 4 schematically illustrates the translation of a virtual ultrasound beam virtually emitted by a virtual ultrasound probe, in accordance with an embodiment;
FIG. 5 schematically illustrates the translation and rotation of a virtual ultrasound beam virtually emitted by a virtual ultrasound probe, in accordance with an embodiment;
FIG. 6 is a block diagram illustrating an embodiment of a system for generating a simulated ultrasound image representing a part of manikin and the tip of a medical tool based on the position of a virtual medical probe; and
FIG. 7 is a block diagram illustrating an exemplary processing module adapted to execute at least some of the steps of the method of FIG. 1A.

It will be noted that throughout the appended drawings, like features are identified by like reference numerals.

### DETAILED DESCRIPTION

The present technology is directed to the generation of simulated medical images such as simulated ultrasound images. The present technology may be used for generating medical images to be displayed in a surgical simulator that may be used to train healthcare practitioners such as surgeon students. The surgical simulator may comprise a manikin on which the healthcare practitioner is to practice a surgical procedure using a medical tool. The manikin may be a training manikin or a medical care manikin. The manikin may be a full body manikin designed to simulate the whole body of a subject for example. Alternatively, the manikin may be a partial body manikin designed to only simulate a portion of a body. Additionally, the surgical simulator may comprise non-humanoid tracking modules for the surgical tools.

Simulated medical images are generated by a simulation engine and then used by the healthcare practitioner to visualize the medical tool within the manikin environment. The simulated medical images of the manikin are generated by a simulation engine according to the position of a virtual ultrasound probe relative to the manikin. A simulated medical image comprises a simulated representation of a part of the subject anatomy as seen by the virtual medical probe, i.e. the simulated representation corresponds to what would be seen on a real medical image taken using a real medical probe. A simulated medical image further comprises a representation of at least the tip of the medical tool when the medical tool has an adequate position relative to the position and orientation of a virtual medical probe, i.e., when at least the tip of the medical tool is comprised within the virtual field of view associated with the virtual medical probe. The virtual field of view corresponds to the field of view that the virtual medical probe would have if the virtual medical probe were real. The position and orientation of the virtual field of view are determined based on the position and orientation of the virtual medical probe. When the virtual medical probe is a medical ultrasound probe, the field of view of the virtual ultrasound probe corresponds to the virtual ultrasound beam of the virtual ultrasound probe and when at least the tip of the medical tool intersects the virtual ultrasound beam, a representation of at least the tip of the medical tool is contained in the simulated ultrasound image. The virtual ultrasound beam corresponds to the ultrasound beam that would have been generated if the virtual ultrasound probe were real. The position and orientation of the virtual ultrasound beam are determined based on the position and orientation of the virtual ultrasound probe.

Since fields of view such as ultrasound beams, whether real or simulated, may be thin, the medical tool may not intersect the virtual field of view and therefore may not appear in the simulated medical images depending on the position and orientation of the virtual probe relative to the position of the medical tool. If the medical tool does not appear on the simulated medical images, the training of the healthcare practitioner is compromised since the healthcare practitioner cannot visualize the position of the manipulated medical tool relative to a target on the virtual patient anatomy. The present technology allows healthcare practitioners to always see the tip of their medical tools within simulated medical images during their training sessions without requiring the presence of an additional person to manipulate medical probes.

Embodiments of the present invention provide for a computer-implemented method for generating a simulated medical image of a virtual patient or manikin part while a user such as a medical practitioner performs a surgical procedure on the virtual patient part using a medical tool or instrument. In surgical procedures, different imaging modalities including soundwave-based modalities or camera-based modalities can be used to visualize the medical tool used in the procedure. For example, for soundwave-based modalities, an ultrasound probe can be used as the medical probe or imaging instrument and, for camera-based modalities, the medical probe or imaging instrument can be a laparoscope or an arthroscope. Arthroscopes are known to be used in surgical procedures involving orthopedic tools while laparoscopes are known to be used in surgical procedures involving laparoscopic tools. Ultrasound probes are known to be used in many surgical procedures as the medical probe or imaging instrument.

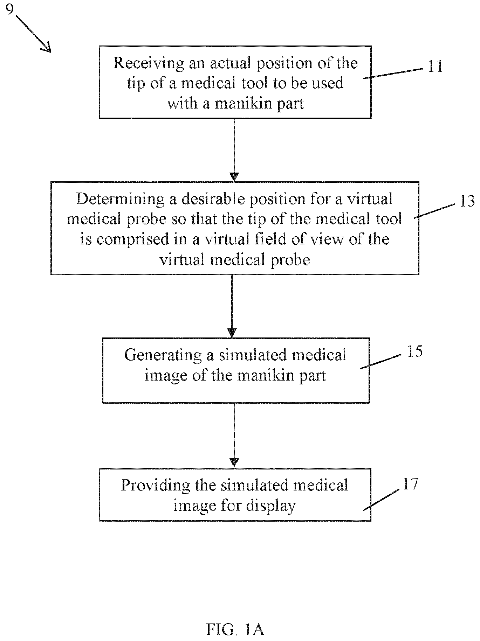

Embodiments of the present invention can be implemented for simulated surgical procedures in which the medical tool used during the procedure needs to be visualized using the medical probe. FIG 1A illustrates one embodiment of a computer-implemented method 9 for generating a simulated medical image to display at least a portion of a manikin part and the tip of a medical tool used in a simulated surgical procedure using a virtual medical probe or imaging instrument. At step 11, the actual position of the tip of a medical tool inserted in a manikin part is received. At step 13, a position for the virtual medical probe is determined in order for the tip of the medical tool inserted in the manikin part to be visible to the user performing the simulated surgical procedure. In this embodiment, a desirable position of the virtual medical probe is determined based on the actual position of the tip of the medical tool. Embodiments of the present invention provide for determining the orientation of the medical tool and determining the desirable position of the medical probe accordingly. The virtual medical probe can be positioned, based on that orientation, such that its virtual field of view intersects the tip of the medical tool, thus providing an unobstructed view of the tip. In an embodiment where the virtual medical probe is a virtual ultrasound probe, the virtual ultrasound probe is positioned such that its virtual ultrasound beam intersects the tip.

In one embodiment, the position of the virtual medical probe may not need to be adjusted after a manipulation of the medical tool by the user in situations where the tip of the medical tool is still within the field of view of the medical virtual probe. In situations where the tip of the medical tool is out of the field of view of the virtual medical probe after a manipulation of the medical tool by the user, the position of the virtual medical probe can be adjusted to keep the tip of the medical tool visible to the user. The adjustment may be dependent on the characteristics of the virtual medical probe. For virtual medical probes mimicking medical probes having a wide field of view such as laparoscopes or arthroscopes, a slight adjustment of the position, orientation or both of the virtual medical probe may suffice to keep the tip visible to the user. For virtual medical probes mimicking medical probes having a narrow field of view, such as ultrasound probes, a greater adjustment of the position, orientation or both of the virtual medical probe may be needed in order to have the narrow field of view intersect the tip of the medical tool.

At step 15 of the method 9, a simulated medical image of the manikin part is generated and, at step 17, the simulated medical image is provided for display to the user. The simulated medical image comprises the manikin part as well as the tip of the medical tool so as to allow the user to have a view of the medical tool being manipulated.

**In** embodiments where the virtual probe may assume a plurality of positions, the desirable position may be selected based on a position of the user or based on an indication provided by the user through an interaction mechanism. For example, if the user is positioned on the right side of the medical tool, the virtual medical probe may be positioned to allow for a view of the manikin part from the opposite perspective, i.e., from the left side of the medical tool to mimic the position that a healthcare professional would have imparted toa real medical probe. The simulation system may be provided with a means to detect the position of the user. Means to detect a position of a user around a structure such as a camera or a presence detector are known to those skilled in the art.

In embodiments where the interaction mechanism is provided between the user and the simulation system, the user may indicate which view is desirable at any moment. For example, the user may indicate a top-down view, a bottom-up view, a left-facing view, a right-facing view or any angled view of the manikin part and tip of the medical tool. In these embodiments, the medical probe is positioned to enable the generation of a medical image presenting the indicated view. The interaction mechanism may be provided in the form of a voice command feature or in other forms known to those skilled in the art.

The flowchart shown in FIG. 1A describes a generic method for generating a simulated medical image using a medical probe such as an ultrasound probe, a laparoscope, an arthroscope, or the like. An ultrasound probe will be used, as an example, for the remaining of the description to illustrate different aspects of the present technology.

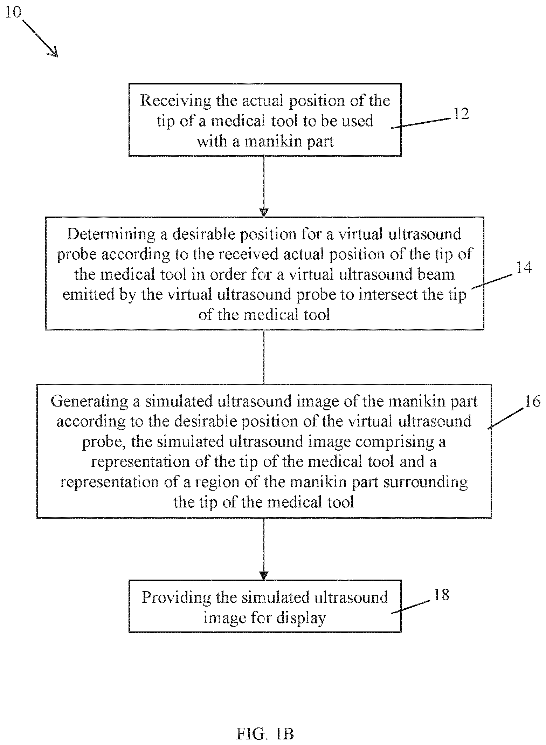

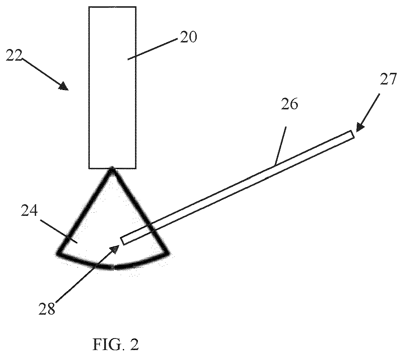

FIG. 1B illustrates one embodiment of a computer-implemented method 10 for generating a simulated ultrasound image of a virtual patient part while a user such as a medical practitioner performs a simulated surgical procedure on the virtual patient part using a medical tool or device. It will be understood that the method 10 is executed by a computer machine provided with at least one processor or processing unit, a memory or storing unit and communication means. FIG. 2 schematically illustrates a virtual ultrasound probe 20 positioned at a position 22 and emitting a virtual ultrasound beam 24, and a medical tool 26 extending between a proximal end 27 and a distal end or tip 28.

At step 12, the position of the tip 28 of the medical tool 26 manipulated by the user while performing the simulated surgical procedure on the virtual patient part is received. In one embodiment, the position of the tip 28 is defined by (x, y, z) coordinates in a reference coordinate system. In another embodiment, the position of the tip 28 is defined relative to a reference point, which may be located on the virtual patient.

In one embodiment, the position of the tip 28 is determined based on the position of another point of the medical tool 26 when the relative position between the tip 28 and the other point is known. For example, the other point may be the proximal end 27 of the medical tool 26. In this case, the step 12 consists in first receiving the position of the other point of the medical tool 26 and then determining the position of the tip 28 based on the received position of the other point of the medical tool 26.

In one embodiment, the method 10 further comprises the step of measuring or determining the position of the tip 28. It will be understood that any adequate method and system for measuring or determining the position of an object or a part of an object may be used for determining the position of the tip 28 of the medical tool 26.

Once the position of the tip 28 has been received at step 12, a target or desirable position 22 for the virtual ultrasound probe 20 is determined based on the received position of the tip 28, at step 14. The desirable position 22 for the virtual ultrasound probe 20 is chosen so that the tip 28 of the medical tool 26 intersects the ultrasound beam 24 associated with the virtual ultrasound probe 26. It will be understood that any adequate method for determining the virtual ultrasound beam 24 emitted by the virtual ultrasound probe 20 according to a given position and orientation of the virtual ultrasound probe 20 may be used.

As described above, the desirable position 22 for the virtual ultrasound probe 20 may be defined as position coordinates in a reference coordinate system and/or the orientation for the virtual ultrasound probe 20. Alternatively, the desirable position 22 for the virtual ultrasound probe 20 may be defined as a displacement and/or a variation in the orientation of the virtual ultrasound probe 20.

In one embodiment, the desirable position 22 for the virtual ultrasound probe 20 is selected amongst predefined positions or predefined position ranges. For example, the desirable position 22 may be selected amongst predefined sets of position coordinates and/or predefined orientations. The predefined positions may also refer to predefined position variations such as predefined variations in position coordinates and/or predefined variations in orientation.

In one embodiment, the desirable position 22 for the virtual ultrasound probe 20 determined at step 14 is chosen so as to be located on a predefined path. In one embodiment, the desirable position 22 may occupy any position along the predefined path. In another embodiment, the desirable position 22 is selected amongst predefined positions all located along the predefined path.

In one embodiment, the desirable position 22 is chosen so that the tip 28 of the medical tool is substantially centered on the virtual ultrasound beam 24, i.e. the tip 28 substantially intersects the central axis or symmetry axis of the virtual ultrasound beam 24 having the shape of a sector of a circle provided with a given thickness, as illustrated in FIG. 2.

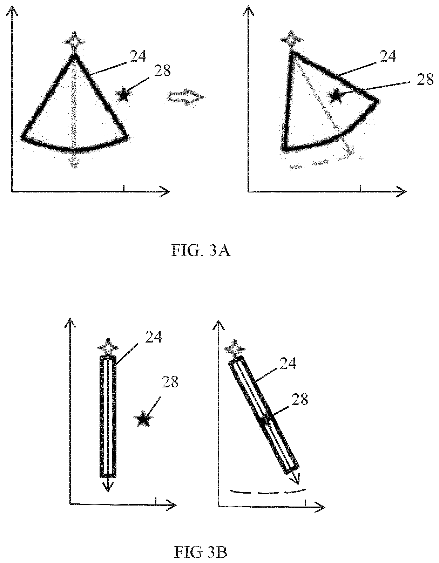

In FIG. 3A, there is provided a first schematic diagram illustrating the tip 28 of the medical tool 26 (not shown) and the virtual ultrasound beam 24 virtually emitted by the virtual ultrasound probe 20 (not shown), wherein the tip 28 does not intersect the virtual ultrasound beam 24 . In this case, step 14 comprises determining a variation in orientation of the virtual ultrasound probe 20 without changing the actual position coordinates of the virtual ultrasound probe 20 so that the virtual ultrasound beam 24 intersects the tip 28 of the medical tool 26. In the present example, and as shown in a second schematic diagram provided in FIG. 3A, the variation in orientation of the virtual prove 20 corresponds to a rotation about an axis orthogonal to the plane of the virtual ultrasound beam 24.

In FIG. 3B, there is provided a first schematic diagram illustrating the tip 28 of the medical tool 26 (not shown) and the virtual ultrasound beam 24 virtually emitted by the virtual ultrasound probe 20, wherein the tip 28 does not intersect the virtual ultrasound beam 24. In this case, step 14 comprises determining a rotation of the virtual ultrasound probe 20 without changing the actual position coordinates of the virtual ultrasound probe 20 so that the virtual ultrasound beam 24 intersects the tip 28 of the medical tool 26. In the present example, and as shown in a second schematic diagram provided in FIG. 3B, the rotation of the virtual ultrasound probe 20 is performed about an axis contained within the plane of the virtual ultrasound beam 24.

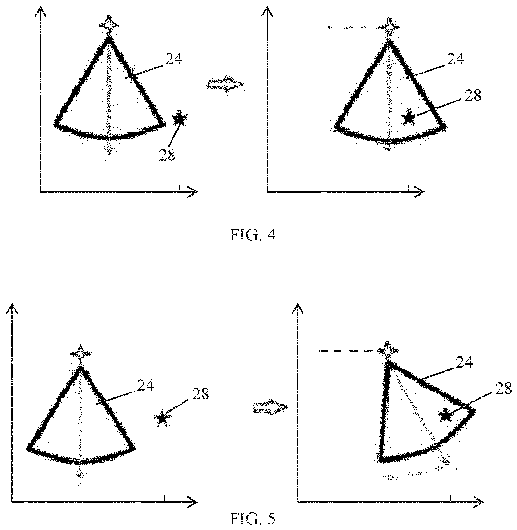

In FIG. 4, there is provided a first schematic diagram illustrating the tip 28 of the medical tool 26 (not shown) and the virtual ultrasound beam 24 virtually emitted by the virtual ultrasound probe 20 (not shown), wherein the tip 28 does not intersect the virtual ultrasound beam 24. In this case, step 14 comprises determining a change in position coordinates for the virtual ultrasound probe 20 without changing the actual orientation of the virtual ultrasound probe 20 so that the virtual ultrasound beam 24 intersects the tip 28. In the present example, the virtual ultrasound probe 20 is translated towards the tip 28 of the medical tool 26, as shown in a second schematic diagram provided in FIG. 4.

In FIG. 5, there is provided a first schematic diagram illustrating the tip 28 of the medical tool 26 (not shown) the virtual ultrasound beam 24 virtually emitted by the virtual ultrasound probe 20 (not shown), wherein the tip 28 does not intersect the virtual ultrasound beam 24. In this case, step 14 comprises determining both a change in position coordinates and a change in orientation for the virtual ultrasound probe 20 so that the virtual ultrasound beam 24 intersects the tip 28. In the present example, the virtual ultrasound probe 20 is translated and oriented towards the tip 28 of the medical tool 26, as shown in a second schematic diagram provided in FIG. 5.

It should be understood that the decision to change the position coordinates of the virtual ultrasound probe 20 only, change the orientation of the virtual ultrasound probe 20 only, or change both the position coordinates and the orientation of the virtual ultrasound probe 20 may be based on predefined rules.

For example, the orientation of the virtual ultrasound probe 20 may be adjusted while its position coordinates remain unchanged when the user wants to see an image based on a pre-defined standardized view (e.g., a mid-esophageal 4 chamber) and/or only wants some adjustment of the orientation so that the displayed anatomy, when following the tip 28, remains similar and coherent to what it expected to be seen in the pre-defined standardized view.

In another example, only the position coordinates of the virtual ultrasound probe 20 are changed when the medical tool 26 is located in a simple anatomical region (e.g., a straight vessel segment) and the expected imaging would not require orientation adjustment (e.g., if the user wants a longitudinal or transverse view to be displayed).

In a further example, both the position and orientation of the virtual ultrasound beam 24 are changed when the medical tool 26 is being displaced over a large distance in a complex anatomy (e.g., with a curved and changing trajectory) and when there is no pre-defined standardized view to act as a starting point for the desired view.

Referring back to FIG. 1B, once the desirable position 22 for the virtual ultrasound probe 20 has been determined at step 14, a simulated ultrasound image is generated at step 16 based on the desirable position 22, i.e., based on the virtual ultrasound beam 24 virtually emitted by the virtual ultrasound probe 20. It will be understood that the virtual ultrasound beam 24 is determined based on the desirable position 22. The generated ultrasound image comprises a representation of a part of the manikin that is intersected by the virtual ultrasound beam 24. Since the desirable position 22 for the virtual ultrasound probe 20 has been chosen so that the tip 28 intersects the virtual ultrasound beam 24, the tip 28 is also represented in the simulated ultrasound image.

It will be understood that any adequate method for creating a simulated ultrasound image in which the representation of the tip of a medical tool is integrated, such as ray casting of a virtual anatomy, may be used at step 16.

At step 18, the simulated ultrasound image is provided for display. **In** one embodiment, the simulated ultrasound image is stored in memory. **In** the same or another embodiment, the simulated ultrasound image is transmitted to a display device for display thereon.

In one embodiment, the method 10 is performed in real time so that the position of the tip 28 of the medical tool 26 is tracked substantially continuously and a desirable position 22 for the virtual ultrasound probe 20 is substantially continuously determined so that the tip 28 appears substantially constantly in the displayed simulated ultrasound images.

In one embodiment, the desirable position 22 for the virtual ultrasound probe is chosen based on the actual position of the medical tool so that the tip 28 of the medical tool 20 is within a given region/section of the simulated ultrasound image. For example, the given region may be a region spaced apart from the edges of the simulated ultrasound region. In another embodiment, the given region may be the central region of the simulated ultrasound image. In one embodiment, the position of the virtual ultrasound probe 20 may remain unchanged in time as long as the tip 28 of the medical tool 26 is located within the given region of the simulated ultrasound image. The position of the virtual ultrasound image is changed only if the tip 28 would not appear within the given region of the simulated ultrasound image. In this case, the position of the virtual ultrasound probe is changed to a new position that allows for the tip 28 to be located within the given region of the simulated ultrasound image.

In one embodiment, the method 10 is used on a per-request basis. For example, the surgical simulator may offer a predefined number of ultrasound views of the manikin, each ultrasound view corresponding to a simulated ultrasound image of the manikin obtained based on a respective and predefined position for the virtual ultrasound probe 20. In this case, the method 10 may be executed only upon request from the user of the surgical simulator. For example, if the medical tool 26 does not appear on any of the predefined ultrasound views, the user may activate the execution of the method 10 so that the tip 28 appears on the displayed simulated ultrasound images. The method 10 may also be used to follow the position of the tip 28. In this case, the method 10 is executed substantially continuously and in real time so that the tip 28 is substantially constantly represented in the displayed simulated ultrasound images.

In one embodiment the method 10 is performed in the context of training for interventional cardiology procedures. In this case, the manikin comprises a heart on which a user trains to perform an interventional cardiology procedure using a medical tool. In this case, the training system may simulate Transesophageal Echocardiography (TEE), i.e. the ultrasound images are generated using a TEE ultrasound probe inserted into the esophagus of a subject. In this case, the method 10 is adapted to generate simulated ultrasound images of a heart according to the position of a virtual TEE ultrasound probe and the position of the virtual TEE ultrasound probe is selected to be located along the esophagus associated with the manikin. In one embodiment, the manikin may be provided with a part or device that mimics a real esophagus. In another embodiment, the esophagus associated with the manikin may be virtual so that the manikin comprises no physical part mimicking a real esophagus. When the esophagus is virtual, the esophagus may be defined as a set of predefined positions, which may be defined relative to the position of the heart for example. The virtual TEE ultrasound probe may take any of the predefined positions at step 14 of the method 10.

In a real interventional cardiology procedure, a surgeon performs the cardiology procedure using a medical tool and an echocardiologist is requested to image the heart of the subject during the cardiology procedure. The echocardiologist introduces a real TEE ultrasound probe into the esophagus of the subject and manipulates the real TEE ultrasound probe so that the distal end of the medical tool manipulated by the surgeon always appears in the displayed ultrasound images. As a result, the surgeon is able to always locate the medical tool relative to the heart during the cardiology procedure.

When it is used to simulate interventional cardiology procedures, the method 10 allows for training a user such as a surgeon student without requiring an echocardiologist to assist the user. The method 10 plays the role of the echocardiologist by automatically adjusting the position of the virtual ultrasound probe 20 so that the tip 28 of the medical tool 26 be continuously represented in the simulated ultrasound images of the heart.

It will be understood that the above-described method 10 may be embodied as computer program product comprising a computer-readable memory storing computer executable instructions thereon that when executed by a computer perform the steps 12-18 of the method 10.

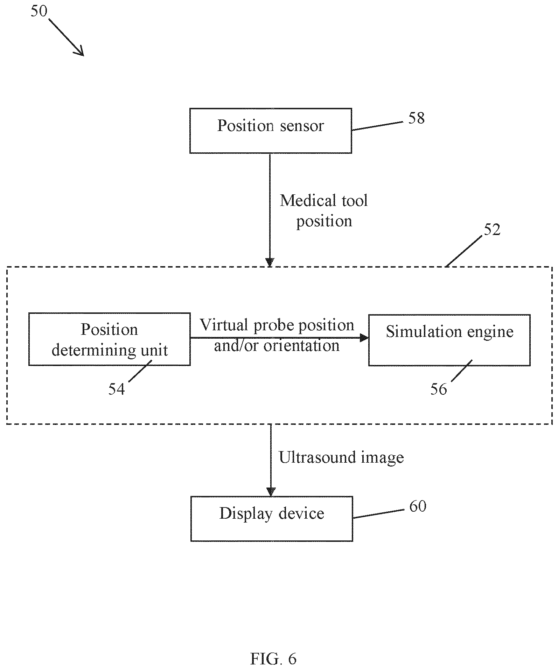

FIG. 6 illustrates one embodiment of a system 50 configured for generating a simulated ultrasound image. The system 50 comprises an image generator 52 comprising a position determining unit 54 and a simulation engine 56, a position sensor 58 and a display device 60. The system 50 may be used with a manikin part that mimics a body part of a subject on which the user of the system is to train to perform a medical procedure using the medical tool 26. For example, the manikin part may comprise a heart when the user trains to perform an interventional cardiology procedure.

The position sensor 58 is configured for determining or measuring the position of the medical tool 26 manipulated by the user of the system 50.

It will be understood that any adequate device/system for measuring the position of the tip 28 of the medical tool 26 manipulated by the user may be used. For example, the position sensor 58 may comprise an optical position tracking system, an electromagnetic position tracking system, an encoder, or the like.

The position sensor 58 may be configured for determining the absolute position of the tip 28 of the medical tool 26. In another embodiment, the position sensor 58 may be configured for determining the position of the tip 28 relative to a reference point which may be located on the manikin part. In this case, the position of the tip 28 corresponds to the position of the tip 28 relative to the manikin part.

In one embodiment, the position sensor 58 is configured for measuring the position of a reference point of the medical tool 26 spaced apart from the tip 28 thereof if the relative position between the reference point and the tip 28 is known. In this case, the position sensor 58 is configured for measuring the position of the reference point of the medical tool 26 and then determining the position of the tip 28 based on the measured position of the reference point.

After determining the position of the tip 28, the position sensor 58 transmits the position of the tip 28 to the image generator 52.

The image generator 52 is configured for generating a simulated ultrasound image based on the received position of the medical tool 26 and transmitting the simulated ultrasound image to the display device 60 for display thereon.

In the illustrated embodiment, the image generator 52 comprises a position determining unit 54 and a simulation engine 56. The position determining unit 54 is configured for receiving the position of the tip 28 from the position sensor 58 and determining a desirable position 22 for the virtual ultrasound probe 20 based on the received position of the tip 28. The desirable position 22 is chosen so that the tip 28 intersects the virtual ultrasound beam 24 associated with the virtual ultrasound probe 20.

In one embodiment, the characteristics of the virtual ultrasound probe 20 such as its shape and dimensions are chosen so as to correspond to the characteristics of a real ultrasound probe so that the virtual ultrasound beam 24 mimics a real ultrasound beam.

In one embodiment, a database stored on a memory contains predefined positions for the virtual ultrasound probe 20. In this case, the position determining unit 54 is configured for selecting the desirable position 22 for the virtual ultrasound probe 20 amongst the predefined positions stored in the database. As described above, a desirable position 22 for the virtual ultrasound probe may refer to desirable position coordinates and/or a desirable orientation for the virtual ultrasound probe, or to a desirable change of position coordinates and/or a desirable change of orientation for the virtual ultrasound probe 20.

In the same or another embodiment, a database stored on a memory contains a predefined range of positions. In this case, the position determining unit 54 is configured for selecting the desirable position 22 so that it is contained in the predefined range of positions.

In one embodiment, a predefined path is stored in memory. The predefined path corresponds to the allowed positions at which the virtual ultrasound probe 20 may be positioned and may be defined as a set of continuous positions or a set of discrete positions. The position determining unit 54 is then configured for determining the desirable position 22 for the virtual ultrasound probe 20 so that the desirable position 22 be located on a predefined path such as along an esophagus. In one embodiment, the virtual ultrasound probe 20 may occupy any position along the predefined path. In another embodiment, the virtual ultrasound probe 20 may only occupy discrete positions along the predefined path.

In one embodiment, the position determining unit 54 is configured for selecting the desirable position 22 for the virtual ultrasound probe 20 so that the tip 28 of the medical tool 26 be substantially centered on the virtual ultrasound beam 24, i.e. the tip 28 substantially intersects the central axis or the symmetry axis of the virtual ultrasound beam 24, as illustrated in FIGS. 3-5.

In one embodiment, the position determining unit 54 is configured for only changing the position coordinates of the virtual ultrasound probe 20. For example, the position determining unit 54 may translate the virtual ultrasound probe 20 so that the virtual ultrasound beam intersects the tip 28, as illustrated in FIG. 4.

In another embodiment, the position determining unit 54 is configured for only changing the orientation of the virtual ultrasound probe 20. For example, the position determining unit 54 may rotate the virtual ultrasound probe 20 so that the virtual ultrasound beam 24 intersects the tip 28 as illustrated in FIG. 3.

In a further embodiment, the position determining unit 54 is configured for changing both the position coordinates and the orientation of the virtual ultrasound probe 20. For example, the position determining unit 54 may translate and rotate the virtual ultrasound probe 20 so that the virtual ultrasound beam 24 intersects the tip 28, as illustrated in FIG. 5.

Once it has been determined, the desirable position 22 for the virtual ultrasound probe 20 is transmitted to the simulation engine 56. The simulation engine 56 is configured for generating a simulated ultrasound image of a part of the manikin based on the received desirable position 22 for the virtual ultrasound probe 20. The simulated ultrasound image comprises a representation of the part of the manikin that is intersected by the virtual ultrasound beam 24 resulting from the desirable position 22 for the virtual ultrasound probe 20. Since the desirable position 22 has been chosen so that the tip 28 of the medical tool 26 intersects the virtual ultrasound beam 24, the simulated ultrasound image further comprises a representation of the tip 28. As a result, the simulated ultrasound image comprises a representation of the tip 28 and a representation of the region of the manikin part that surrounds the tip 28.

It will be understood that the simulation engine 56 may use any adequate method for generating a simulated ultrasound image in which the representation of the tip 28 is integrated.

In an embodiment in which the simulation engine 56 only receives desirable position coordinates or a desirable change of position coordinates for the virtual ultrasound probe 20, the simulation engine 56 considers that the orientation of the virtual ultrasound probe 20 remains unchanged and uses the previous orientation of the virtual ultrasound probe 20 along with the received desirable position coordinates or the received desirable change of position coordinates for the virtual ultrasound probe 20 to generate the virtual image.

In an embodiment in which the simulation engine 56 only receives a desirable orientation for the virtual probe 20, the simulation engine 56 considers that the position coordinates of the virtual ultrasound probe remain unchanged and uses the previous position coordinates of the virtual ultrasound probe 20 along with the received desirable orientation for the virtual ultrasound probe 20 to generate the virtual ultrasound image.

In an embodiment in which the desirable position 22 received by the simulation engine 56 comprises bother desirable position coordinates and a desirable orientation, simulation engine 56 uses the received desirable position coordinates and received desirable orientation for the virtual ultrasound probe 20 to generate the virtual ultrasound image.

After generating the simulated ultrasound image, the simulation engine 56 transmits the simulated ultrasound image to the display device 60. The display device 60 then displays the simulated ultrasound image thereon. Therefore, as the user of the system 50 moves the medical tool 26, the simulated ultrasound image always contain a representation of the medical tool 26, allowing the user to visualize the tip 28 of the medical tool 26 relative to manikin.

In one embodiment, the system 50 is further configured for offering preselected ultrasound views of the manikin part. Each preselected ultrasound view corresponds to a simulated ultrasound image of the manikin obtained based on a respective and predefined position of the virtual ultrasound probe 20. In this case, the user inputs a command indicative of a desired preselected view and the simulation engine 56 generates the simulated ultrasound image based on the position of the virtual ultrasound probe 20 associated with the desired preselected view upon receipt of the command. It will be understood that the simulated ultrasound image may comprise a representation of the medical tool 26 if the medical tool 26 intersects the virtual ultrasound beam 24 generated according to the position of the virtual ultrasound probe 20 associated with the desired preselected view.

In one embodiment, an initial position for the virtual ultrasound probe 20 is stored in memory. When the system 50 starts being operated by the user, the initial position for the virtual ultrasound probe 20 is retrieved from the memory by the position determining unit 54 and the simulation engine 56 generates the first simulated ultrasound image to be displayed based on the initial position for the virtual ultrasound probe 20. In one embodiment such as when the system 50 may be used for training a user in a plurality of surgical procedures, a plurality of initial positions for the virtual ultrasound probe 20 may be stored in memory. Each surgical procedure may have a respective initial position for the virtual ultrasound probe 20 associated thereto. In this case, before using the system 50, the user inputs a desired surgical procedure via a user interface such as a voice command system and the position determining unit 54 retrieves the initial position for the virtual ultrasound probe 20 based on the selected surgical procedure.

In one embodiment, the initial position for the virtual ultrasound probe 20 is not predefined and stored in a memory, but rather determined by the position determining unit 54 based on user preferences. In this case, the user inputs user preferences via a user interface such as a voice command system.

The user may also input a command indicative of a tracking mode. In the tracking mode, the image generator 52 operates as described above, i.e. the image generator 52 determines a desirable position 22 for the virtual ultrasound probe 20 allowing the tip 28 of the medical tool 26 to intersect the virtual ultrasound beam 24 so that the tip 28 tool is represented in the simulated ultrasound image. The tracking tool allows for the tip 28 to always be represented in the simulated ultrasound images, thereby allowing the user of the system 50 to continuously monitor the position of the medical tool 26 relative to the manikin while looking at the displayed simulated ultrasound images.

Although the system illustrated in FIG. 6 is configured to simulate ultrasound imaging, those skilled in the art will recognize that the system can be adapted for other types of medical imaging such as laparoscope imaging or arthroscope imaging.

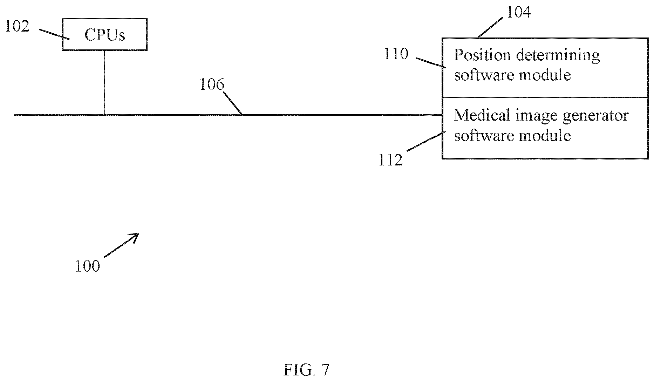

FIG. 7 is a block diagram illustrating an exemplary processing module 100 for executing the steps 12 to 18 of the method 10, in accordance with some embodiments. The processing module 100 typically includes one or more Computer Processing Units (CPUs) and/or Graphic Processing Units (GPUs) 102 for executing software modules or programs and/or instructions stored in memory 104 and thereby performing processing operations, memory 104, and one or more communication buses 106 for interconnecting these components. The communication buses 106 optionally include circuitry (sometimes called a chipset) that interconnects and controls communications between system components. The memory 104 includes high-speed random-access memory, such as DRAM, SRAM, DDR RAM or other random-access solid state memory devices, and may include non-volatile memory, such as one or more magnetic disk storage devices, optical disk storage devices, flash memory devices, or other non-volatile solid state storage devices. The memory 104 optionally includes one or more storage devices remotely located from the CPU(s) 102. The memory 104, or alternately the non-volatile memory device(s) within the memory 104, comprises a non-transitory computer readable storage medium. In some embodiments, the memory 104, or the computer readable storage medium of the memory 104 stores the following programs, software modules, and data structures, or a subset thereof:
a position determining software module 110 for receiving the position of the tip 28 of a medical tool 26 and determining a desirable position 22 for a virtual ultrasound probe 20, as described above; and
a medical image generator software module 112 for generating a simulated medical image of a manikin based on the desirable position 22 and providing the generated simulated medical image for display.

Each of the above identified elements may be stored in one or more of the previously mentioned memory devices and corresponds to a set of instructions for performing a function described above. The above identified software modules or programs (i.e., sets of instructions) need not be implemented as separate software programs, procedures or software modules, and thus various subsets of these software modules may be combined or otherwise re-arranged in various embodiments. In some embodiments, the memory 104 may store a subset of the software modules and data structures identified above. Furthermore, the memory 104 may store additional modules and data structures not described above.

The schematic block diagram shown in FIG. 7 is intended to provide an exemplary functional view of the various features. In practice, and as recognized by the person skilled in the art, items shown separately could be combined and some items could be separated. Those skilled in the art will recognize that the processing module shown in FIG. 7 can also be adapted for implementation using any adequate medical probe such as a laparoscope or an arthroscope.

The embodiments of the invention described above are intended to be exemplary only. The scope of the invention is therefore intended to be limited solely by the scope of the appended claims.

## Claims

1. A computer-implemented method for generating a simulated medical image of a manikin part, the computer-implemented method comprising:
determining an actual position of a tip (28) of a medical tool (26);
determining a desirable position for a virtual medical probe (20) based on the actual position of the tip (28) of the medical tool (26) in order for a virtual field of view of the virtual medical probe to intersect the tip of the medical tool;
generating the simulated medical image of the manikin part according to the desirable position of the virtual medical probe (20), the simulated medical image comprising a representation of the tip (28) of the medical tool (26) and a representation of a region of the manikin part surrounding the tip of the medical tool; and
providing the simulated medical image for display.

2. The computer-implemented method of claim 1, wherein the virtual medical probe (20) comprises a virtual ultrasound probe, the virtual field of view of the virtual medical probe (20) comprises a virtual ultrasound beam emitted by the virtual ultrasound probe, and the simulated medical image comprises a simulated ultrasound image.

3. The computer-implemented method of claim 2, wherein said determining the desirable position for the virtual ultrasound probe (20) comprises at least one of determining desirable position coordinates for a reference point located on the virtual ultrasound probe (20) and determining a desirable orientation of the virtual ultrasound probe.

4. The computer-implemented method of claim 3, wherein said determining the desirable position for the virtual ultrasound probe (20) comprises determining the desirable orientation of the virtual ultrasound probe (20), the desirable position coordinates for the reference point located on the virtual ultrasound probe (20) being fixed and said determining the desirable orientation for the virtual ultrasound probe (20) preferably comprising selecting the desirable orientation for the virtual ultrasound probe (20) amongst predefined orientations based on the actual position of the tip (28) of the medical tool (26).

5. The computer-implemented method of claim 3, wherein said determining the desirable position for the virtual ultrasound probe (20) comprises determining the desirable position coordinates for the reference point located on the virtual ultrasound probe (20), the desirable orientation of the virtual ultrasound probe (20) being fixed, said determining the desirable position coordinates for the reference point located on the virtual ultrasound probe (20) preferably comprising selecting the desirable position coordinates amongst predefined position coordinates based on the position of the tip (28) of the medical tool (26) and the manikin part preferably comprising an esophagus, the predefined position coordinates being located along the esophagus.

6. The computer-implemented method of claim 2, wherein said determining the desirable position for the virtual ultrasound probe (20) comprises determining a desirable position variation for the virtual ultrasound probe (20), said determining the desirable position variation for the virtual ultrasound probe (20) preferably comprising at least one of determining a desirable variation in position coordinates for a reference point located on the virtual ultrasound probe (20) and determining a desirable variation in orientation of the virtual ultrasound probe (20).

7. The computer-implemented method of claim 1, wherein the virtual medical probe (20) comprises one of a virtual arthroscope and a virtual laparoscope.

8. A system for generating a simulated medical image of a manikin part, the system comprising:
a position sensor for determining an actual position of a tip (28) of a medical tool (26);
a probe determining unit for determining a desirable position for a virtual medical probe (20) based on the actual position of the tip (28) of the medical tool (26) in order for a virtual field of view of the virtual medical probe (20) to intersect the tip (28) of the medical tool (26); and
a simulation engine for generating the simulated medical image of the manikin part according to the desirable position of the virtual medical probe (20), and providing the simulated medical image for display, the simulated medical image comprising a representation of the tip (28) of the medical tool (26) and a representation of a region of the manikin part surrounding the tip (28) of the medical tool (26).

9. The system of claim 8, wherein the virtual medical probe (20) comprises a virtual ultrasound probe, the virtual field of view of the virtual medical probe (20) comprises a virtual ultrasound beam emitted by the virtual ultrasound probe (20), and the simulated medical image comprises a simulated ultrasound image.

10. The system of claim 9, wherein the probe determining unit is configured for determining the desirable position by determining at least one of desirable position coordinates for a reference point located on the virtual ultrasound probe (20) and a desirable orientation of the virtual ultrasound probe (20).

11. The system of claim 10, wherein the probe determining unit is configured for determining the desirable position by determining the desirable orientation of the virtual ultrasound probe (20), the desirable position coordinates for the reference point located on the virtual ultrasound probe (20) being fixed and the probe determining unit being preferably configured for selecting the desirable orientation for the virtual ultrasound probe (20) amongst predefined orientations based on the actual position of the tip (28) of the medical tool (26).

12. The system of claim 10, wherein the probe determining unit is configured for determining the desirable position coordinates for the reference point located on the virtual ultrasound probe (20), the desirable orientation of the virtual ultrasound probe (20) being fixed, the probe determining unit being preferably configured for selecting the desirable position coordinates amongst predefined position coordinates based on the position of the tip of the medical tool and the manikin part comprising preferably an esophagus, the predefined position coordinates being located along the esophagus.

13. The system of claim 9, wherein the probe determining unit is configured for determining a desirable position variation for the virtual ultrasound probe (20), the probe determining unit being preferably configured for at least one of determining a desirable variation in position coordinates for a reference point located on the virtual ultrasound probe (20) and determining a desirable variation in orientation of the virtual ultrasound probe (20).

14. The system of claim 8, wherein the virtual medical probe comprises one of a virtual arthroscope and a virtual laparoscope.

15. A computer program product for generating a simulated medical image of a manikin part, the computer program product comprising a computer readable memory storing computer executable instructions thereon that when executed by a computer perform the method steps of:
determining a desirable position for a virtual medical probe (20) based on an actual position of a tip (28) of a medical tool (26) in order for a virtual field of view of the virtual medical probe (20) to intersect the tip (28) of the medical tool (26);
generating the simulated medical image of the manikin part according to the desirable position of the virtual medical probe (20), the simulated medical image comprising a representation of the tip (28) of the medical tool (26) and a representation of a region of the manikin part surrounding the tip (28) of the medical tool (26); and
providing the simulated ultrasound image for display.

## Patentansprüche

1. Computerimplementiertes Verfahren zum Erzeugen eines simulierten medizinischen Bildes eines Teils des Menschmodells, das computerimplementierte Verfahren umfassend:
Bestimmen der tatsächlichen Position einer Spitze (28) eines medizinischen Werkzeugs (26);
Bestimmen einer gewünschten Position für eine virtuelle medizinische Sonde (20) basierend auf der tatsächlichen Position der Spitze (28) des medizinischen Werkzeugs (26), damit ein virtuelles Sichtfeld der virtuellen medizinischen Sonde die Spitze des medizinischen Werkzeugs schneidet;
Erzeugen des simulierten medizinischen Bildes des Teils des Menschmodells entsprechend der gewünschten Position der virtuellen medizinischen Sonde (20), das simulierte medizinische Bild umfassend eine Darstellung der Spitze (28) des medizinischen Werkzeugs (26) und eine Darstellung einer Region des Teils des Menschmodells, die die Spitze des medizinischen Werkzeugs umgibt; und
Bereitstellen des simulierten medizinischen Bildes zur Anzeige.

2. Computerimplementiertes Verfahren nach Anspruch 1, wobei die virtuelle medizinische Sonde (20) eine virtuelle Ultraschallsonde umfasst, das virtuelle Sichtfeld der virtuellen medizinischen Sonde (20) einen von der virtuellen Ultraschallsonde emittierten virtuellen Ultraschallstrahl umfasst und das simulierte medizinische Bild ein simuliertes Ultraschallbild umfasst.

3. Computerimplementiertes Verfahren nach Anspruch 2, wobei das Bestimmen der gewünschten Position für die virtuelle Ultraschallsonde (20) mindestens das Bestimmen von Koordinaten der gewünschten Position für einen Referenzpunkt, der sich auf der virtuellen Ultraschallsonde (20) befindet, und das Bestimmen einer gewünschten Ausrichtung der virtuellen Ultraschallsonde umfasst.

4. Computerimplementiertes Verfahren nach Anspruch 3, wobei das Bestimmen der gewünschten Position für die virtuelle Ultraschallsonde (20) das Bestimmen der gewünschten Ausrichtung der virtuellen Ultraschallsonde (20) umfasst, die gewünschten Positionskoordinaten für den Referenzpunkt, der sich auf der virtuellen Ultraschallsonde (20) befindet, festgelegt sind und das Bestimmen der gewünschten Ausrichtung für die virtuelle Ultraschallsonde (20) vorzugsweise das Auswählen der gewünschten Ausrichtung für die virtuelle Ultraschallsonde (20) aus vordefinierten Ausrichtungen basierend auf der tatsächlichen Position der Spitze (28) des medizinischen Werkzeugs (26) umfasst.

5. Computerimplementiertes Verfahren nach Anspruch 3, wobei das Bestimmen der gewünschten Position für die virtuelle Ultraschallsonde (20) das Bestimmen der gewünschten Positionskoordinaten für den Referenzpunkt umfasst, der sich auf der virtuellen Ultraschallsonde (20) befindet, wobei die gewünschte Ausrichtung der virtuellen Ultraschallsonde (20) festgelegt ist, das Bestimmen der gewünschten Positionskoordinaten für den Referenzpunkt, der sich auf der virtuellen Ultraschallsonde (20) befindet, vorzugsweise das Auswählen der gewünschten Positionskoordinaten aus vordefinierten Positionskoordinaten basierend auf der Position der Spitze (28) des medizinischen Werkzeugs (26) umfasst und das Teil des Menschmodells vorzugsweise eine Speiseröhre umfasst, wobei sich die vordefinierten Positionskoordinaten entlang der Speiseröhre befinden.

6. Computerimplementiertes Verfahren nach Anspruch 2, wobei das Bestimmen der gewünschten Position für die virtuelle Ultraschallsonde (20) das Bestimmen einer gewünschten Positionsänderung für die virtuelle Ultraschallsonde (20) umfasst, das Bestimmen der gewünschten Positionsänderung für die virtuelle Ultraschallsonde (20) vorzugsweise mindestens das Bestimmen einer gewünschten Änderung der Positionskoordinaten für einen Referenzpunkt, der sich auf der virtuellen Ultraschallsonde (20) befindet, und das Bestimmen einer gewünschten Änderung der Ausrichtung der virtuellen Ultraschallsonde (20) umfasst.

7. Computerimplementiertes Verfahren nach Anspruch 1, wobei die virtuelle medizinische Sonde (20) eines von einem virtuellen Arthroskop und einem virtuellen Laparoskop umfasst.

8. System zur Erzeugung eines simulierten medizinischen Bildes eines Teils des Menschmodells, das System umfassend:
einen Positionssensor zum Bestimmen einer tatsächlichen Position einer Spitze (28) eines medizinischen Werkzeugs (26);
eine Sondenbestimmungseinheit zum Bestimmen einer gewünschten Position für eine virtuelle medizinische Sonde (20) basierend auf der tatsächlichen Position der Spitze (28) des medizinischen Werkzeugs (26), damit ein virtuelles Sichtfeld der virtuellen medizinischen Sonde (20) die Spitze (28) des medizinischen Werkzeugs (26) schneidet; und
eine Simulations-Engine zum Erzeugen des simulierten medizinischen Bildes des Teils des Menschmodells gemäß der gewünschten Position der virtuellen medizinischen Sonde (20) und Bereitstellen des simulierten medizinischen Bildes zur Anzeige, das simulierte medizinische Bild umfassend eine Darstellung der Spitze (28) des medizinischen Werkzeugs (26) und eine Darstellung einer Region des Teils des Menschmodells, die die Spitze (28) des medizinischen Werkzeugs (26) umgibt.

9. System nach Anspruch 8, wobei die virtuelle medizinische Sonde (20) eine virtuelle Ultraschallsonde umfasst, das virtuelle Sichtfeld der virtuellen medizinischen Sonde (20) einen von der virtuellen Ultraschallsonde (20) ausgesendeten virtuellen Ultraschallstrahl umfasst und das simulierte medizinische Bild ein simuliertes Ultraschallbild umfasst.

10. System nach Anspruch 9, wobei die Sondenbestimmungseinheit so konfiguriert ist, dass sie die gewünschte Position bestimmt, indem sie mindestens eine der gewünschten Positionskoordinaten für einen Referenzpunkt, der sich auf der virtuellen Ultraschallsonde (20) befindet, und eine gewünschte Ausrichtung der virtuellen Ultraschallsonde (20) bestimmt.

11. System nach Anspruch 10, wobei die Sondenbestimmungseinheit so konfiguriert ist, dass sie die gewünschte Position durch Bestimmen der gewünschten Ausrichtung der virtuellen Ultraschallsonde (20) bestimmt, die gewünschten Positionskoordinaten für den Referenzpunkt, der sich auf der virtuellen Ultraschallsonde (20) befindet, festgelegt sind und die Sondeneinheit vorzugsweise so konfiguriert ist, dass sie die gewünschte Ausrichtung für die virtuelle Ultraschallsonde (20) aus vordefinierten Ausrichtungen basierend auf der tatsächlichen Position der Spitze (28) des medizinischen Werkzeugs (26) auswählt.

12. System nach Anspruch 10, wobei die Sondenbestimmungseinheit so konfiguriert ist, dass sie die gewünschten Positionskoordinaten für den Referenzpunkt auf der virtuellen Ultraschallsonde (20) bestimmt, wobei die gewünschte Ausrichtung der virtuellen Ultraschallsonde (20) festgelegt ist, die Sondenbestimmungseinheit vorzugsweise so konfiguriert ist, dass sie die gewünschten Positionskoordinaten aus vordefinierten Positionskoordinaten basierend auf der Position der Spitze des medizinischen Werkzeugs und des Teils des Menschmodells, vorzugsweise umfassend eine Speiseröhre, auswählt, wobei sich die vordefinierten Positionskoordinaten entlang der Speiseröhre befinden.

13. System nach Anspruch 9, wobei die Sondenbestimmungseinheit so konfiguriert ist, dass sie eine gewünschte Positionsänderung für die virtuelle Ultraschallsonde (20) bestimmt, die Sondenbestimmungseinheit vorzugsweise so konfiguriert ist, dass sie mindestens das Bestimmen einer gewünschten Änderung der Positionskoordinaten für einen auf der virtuellen Ultraschallsonde (20) befindlichen Referenzpunkt und das Bestimmen einer gewünschten Änderung der Ausrichtung der virtuellen Ultraschallsonde (20) ermöglicht.

14. System nach Anspruch 8, wobei die virtuelle medizinische Sonde eines von einem virtuellen Arthroskop und einem virtuellen Laparoskop umfasst.

15. Computerprogrammprodukt zum Erzeugen eines simulierten medizinischen Bildes eines Teils des Menschmodells, das Computerprogrammprodukt umfassend einen computerlesbaren Speicher, auf dem computerausführbare Anweisungen gespeichert sind, die bei Ausführung durch einen Computer die folgenden Verfahren ausführen:
Bestimmen einer gewünschten Position für eine virtuelle medizinische Sonde (20) basierend auf einer tatsächlichen Position einer Spitze (28) eines medizinischen Werkzeugs (26), damit ein virtuelles Sichtfeld der virtuellen medizinischen Sonde (20) die Spitze (28) des medizinischen Werkzeugs (26) schneidet;
Erzeugen des simulierten medizinischen Bildes des Teils des Menschmodells gemäß der gewünschten Position der virtuellen medizinischen Sonde (20), das simulierte medizinische Bild umfassend eine Darstellung der Spitze (28) des medizinischen Werkzeugs (26) und eine Darstellung einer Region des Teils des Menschmodells, die die Spitze (28) des medizinischen Werkzeugs (26) umgibt; und
Bereitstellen des simulierten Ultraschallbildes zur Anzeige.

## Revendications

1. Procédé mis en œuvre par ordinateur pour la génération d'une image médicale simulée d'une partie de mannequin, le procédé mis en œuvre par ordinateur comprenant :
la détermination d'une position réelle d'une pointe (28) d'un outil médical (26) ;
la détermination d'une position souhaitée pour une sonde médicale virtuelle (20) sur la base de la position réelle de la pointe (28) de l'outil médical (26) afin qu'un champ de vision virtuel de la sonde médicale virtuelle croise la pointe de l'outil médical ;
la génération de l'image médicale simulée de la partie de mannequin selon la position souhaitée de la sonde médicale virtuelle (20), l'image médicale simulée comprenant une représentation de la pointe (28) de l'outil médical (26) et une représentation d'une région de la partie de mannequin entourant la pointe de l'outil médical ; et
la fourniture de l'image médicale simulée à des fins d'affichage.

2. Procédé mis en œuvre par ordinateur selon la revendication 1, dans lequel la sonde médicale virtuelle (20) comprend une sonde à ultrasons virtuelle, le champ de vision virtuel de la sonde médicale virtuelle (20) comprend un faisceau ultrasonore virtuel émis par la sonde à ultrasons virtuelle, et l'image médicale simulée comprend une image ultrasonore simulée.

3. Procédé mis en œuvre par ordinateur selon la revendication 2, dans lequel ladite détermination de la position souhaitée pour la sonde à ultrasons virtuelle (20) comprend au moins l'une parmi la détermination de coordonnées de position souhaitées pour un point de référence situé sur la sonde à ultrasons virtuelle (20) et la détermination d'une orientation souhaitée de la sonde à ultrasons virtuelle.

4. Procédé mis en œuvre par ordinateur selon la revendication 3, dans lequel ladite détermination de la position souhaitée pour la sonde à ultrasons virtuelle (20) comprend la détermination de l'orientation souhaitée de la sonde à ultrasons virtuelle (20), les coordonnées de position souhaitées pour le point de référence situé sur la sonde à ultrasons virtuelle (20) étant fixées et ladite détermination de l'orientation souhaitée pour la sonde à ultrasons virtuelle (20) comprenant de préférence la sélection de l'orientation souhaitée pour la sonde à ultrasons virtuelle (20) parmi des orientations prédéfinies, sur la base de la position réelle de la pointe (28) de l'outil médical (26).

5. Procédé mis en œuvre par ordinateur selon la revendication 3, dans lequel ladite détermination de la position souhaitée pour la sonde à ultrasons virtuelle (20) comprend la détermination des coordonnées de position souhaitées pour le point de référence situé sur la sonde à ultrasons virtuelle (20), l'orientation souhaitée de la sonde à ultrasons virtuelle (20) étant fixe, ladite détermination des coordonnées de position souhaitées pour le point de référence situé sur la sonde à ultrasons virtuelle (20) comprenant de préférence la sélection des coordonnées de position souhaitées parmi des coordonnées de position prédéfinies, sur la base de la position de la pointe (28) de l'outil médical (26), et la partie de mannequin comprenant de préférence un œsophage, les coordonnées de position prédéfinies étant situées le long de l'œsophage.

6. Procédé mis en œuvre par ordinateur selon la revendication 2, dans lequel ladite détermination de la position souhaitée pour la sonde à ultrasons virtuelle (20) comprend la détermination d'une variation de position souhaitée pour la sonde à ultrasons virtuelle (20), ladite détermination de la variation de position souhaitée pour la sonde à ultrasons virtuelle (20) comprenant de préférence au moins l'une parmi la détermination d'une variation souhaitée de coordonnées de position pour un point de référence situé sur la sonde à ultrasons virtuelle (20) et la détermination d'une variation souhaitée d'orientation de la sonde à ultrasons virtuelle (20).

7. Procédé mis en œuvre par ordinateur selon la revendication 1, dans lequel la sonde médicale virtuelle (20) comprend l'un parmi un arthroscope virtuel et un laparoscope virtuel.

8. Système de génération d'une image médicale simulée d'une partie de mannequin, le système comprenant :
un capteur de position pour déterminer une position réelle d'une pointe (28) d'un outil médical (26) ;
une unité de détermination de sonde pour déterminer une position souhaitée pour une sonde médicale virtuelle (20) sur la base de la position réelle de la pointe (28) de l'outil médical (26) afin qu'un champ de vision virtuel de la sonde médicale virtuelle (20) croise la pointe (28) de l'outil médical (26) ; et
un moteur de simulation pour générer l'image médicale simulée de la partie de mannequin selon la position souhaitée de la sonde médicale virtuelle (20), et fournir l'image médicale simulée à des fins d'affichage, l'image médicale simulée comprenant une représentation de la pointe (28) de l'outil médical (26) et une représentation d'une région de la partie de mannequin entourant la pointe (28) de l'outil médical (26).

9. Système selon la revendication 8, dans lequel la sonde médicale virtuelle (20) comprend une sonde à ultrasons virtuelle, le champ de vision virtuel de la sonde médicale virtuelle (20) comprend un faisceau ultrasonore virtuel émis par la sonde à ultrasons virtuelle (20), et l'image médicale simulée comprend une image ultrasonore simulée.

10. Système selon la revendication 9, dans lequel l'unité de détermination de sonde est configurée pour déterminer la position souhaitée en déterminant au moins l'une parmi des coordonnées de position souhaitées pour un point de référence situé sur la sonde à ultrasons virtuelle (20) et une orientation souhaitée de la sonde à ultrasons virtuelle (20).

11. Système selon la revendication 10, dans lequel l'unité de détermination de sonde est configurée pour déterminer la position souhaitée en déterminant l'orientation souhaitée de la sonde à ultrasons virtuelle (20), les coordonnées de position souhaitées pour le point de référence situé sur la sonde à ultrasons virtuelle (20) étant fixes et l'unité de détermination de sonde étant de préférence configurée pour sélectionner l'orientation souhaitée pour la sonde à ultrasons virtuelle (20) parmi des orientations prédéfinies, sur la base de la position réelle de la pointe (28) de l'outil médical (26).

12. Système selon la revendication 10, dans lequel l'unité de détermination de sonde est configurée pour déterminer les coordonnées de position souhaitées pour le point de référence situé sur la sonde à ultrasons virtuelle (20), l'orientation souhaitée de la sonde à ultrasons virtuelle (20) étant fixe, l'unité de détermination de sonde étant de préférence configurée pour sélectionner les coordonnées de position souhaitées parmi les coordonnées de position prédéfinies, sur la base de la position de la pointe de l'outil médical et la partie de mannequin comprenant de préférence un œsophage, les coordonnées de position prédéfinies étant situées le long de l'œsophage.

13. Système selon la revendication 9, dans lequel l'unité de détermination de sonde est configurée pour déterminer une variation de position souhaitée pour la sonde à ultrasons virtuelle (20), l'unité de détermination de sonde étant de préférence configurée pour au moins l'une parmi la détermination d'une variation souhaitée de coordonnées de position pour un point de référence situé sur la sonde à ultrasons virtuelle (20) et la détermination d'une variation souhaitée d'orientation de la sonde à ultrasons virtuelle (20).

14. Système selon la revendication 8, dans lequel la sonde médicale virtuelle comprend l'un parmi un arthroscope virtuel et un laparoscope virtuel.

15. Produit programme d'ordinateur pour générer une image médicale simulée d'une partie de mannequin, le produit programme d'ordinateur comprenant une mémoire lisible par ordinateur stockant des instructions exécutables par ordinateur sur celle-ci qui, lorsqu'elles sont exécutées par un ordinateur, réalisent les étapes de procédé suivantes :
la détermination d'une position souhaitée pour une sonde médicale virtuelle (20) sur la base d'une position réelle d'une pointe (28) d'un outil médical (26) afin qu'un champ de vision virtuel de la sonde médicale virtuelle (20) croise la pointe (28) de l'outil médical (26) ;
la génération de l'image médicale simulée de la partie de mannequin selon la position souhaitée de la sonde médicale virtuelle (20), l'image médicale simulée comprenant une représentation de la pointe (28) de l'outil médical (26) et une représentation d'une région de la partie de mannequin entourant la pointe (28) de l'outil médical (26) ; et
la fourniture de l'image ultrasonore simulée à des fins d'affichage.
